# EUROPEAN PATENT SPECIFICATION

(11) **EP 3 234 164 B1**
(45) Date of publication and mention of the grant of the patent: **13.03.2019**
(21) Application number: 15823596.0
(22) Date of filing: 09.12.2015
(51) Int. Cl.: C12P 7/22, C12N 9/02

(54) **A METHOD FOR THE ENZYMATIC CONVERSION OF A PHENOL SUBSTRATE INTO A CORRESPONDING CATECHOL PRODUCT**
VERFAHREN ZUR ENZYMATISCHEN UMWANDLUNG EINES PHENOLSUBSTRATS IN EIN ENTSPRECHENDES CATECHOLPRODUKT
PROCÉDÉ POUR LA CONVERSION ENZYMATIQUE D'UN SUBSTRAT PHÉNOLIQUE EN UN PRODUIT CATÉCHOL CORRESPONDANT

(30) Priority: 17.12.2014 GB 201422508
(43) Date of publication of application: 25.10.2017
(73) Proprietor: University College Dublin National University Of Ireland, Dublin, Dublin 4 (IE)
(72) Inventor: O'CONNOR, Kevin, Dublin 4 (IE); MOLLOY, Susan, Dublin 4 (IE); DAVIS, Reeta, Dublin 4 (IE); SHAW, Wesley, Dublin 4 (IE)
(74) Representative: Cahill, Susanne
(86) International application number: PCT/EP2015/079177
(87) International publication number: WO 2016/096579

(56) References cited:
- EP-A1- 1 310 562
- BROOKS S J ET AL: "Tyrosol to hydroxytyrosol biotransformation by immobilised cell extracts of Pseudomonas putida F6", ENZYME AND MICROBIAL TECHNOLOGY, vol. 39, no. 2, 26 June 2006 (2006-06-26), pages 191-196, XP027948987, STONEHAM, MA, US ISSN: 0141-0229, DOI: 10.1016/J.ENZMICTEC.2005.10.025 [retrieved on 2006-06-26]
- S. J. BROOKS ET AL: "Biotransformation of halophenols using crude cell extracts of Pseudomonas putida F6", APPLIED MICROBIOLOGY AND BIOTECHNOLOGY, vol. 64, 2004, pages 486-492, XP002755254,
- MOLLOY, S. ET AL: "ENGINEERING OF A BACTERIAL TYROSINASE FOR IMPROVED CATALYTIC EFFICIENCY TOWARDS D-TYROSINE USING RANDOM AND SITE DIRECTED MUTAGENESIS APPROACHES.", BIOTECHNOLOGY AND BIOENGINEERING, vol. 110, no. 7, 22 July 2013 (2013-07-22) , pages 1849-1857, XP002755255, DOI: 10.1002/BIT.24859 cited in the application
- MICHAEL FAIRHEAD ET AL: "Bacterial tyrosinases: old enzymes with new relevance to biotechnology", NEW BIOTECHNOLOGY, vol. 29, no. 2, January 2012 (2012-01), pages 183-191, XP028438595, ISSN: 1871-6784, DOI: 10.1016/J.NBT.2011.05.007 [retrieved on 2011-05-31]
- ESPIN J C ET AL: "SYNTHESIS OF THE ANTIOXIDANT HYDROXYTYROSOL USING TYROSINASE AS BIOCATALYST", JOURNAL OF AGRICULTURAL AND FOOD CHEMISTRY, vol. 49, no. 3, 1 January 2001 (2001-01-01), pages 1187-1193, XP001083568, AMERICAN CHEMICAL SOCIETY, US ISSN: 0021-8561, DOI: 10.1021/JF001258B
- D. HERNANDEZ-ROMERO ET AL: "Polyphenol Oxidase Activity Expression in Ralstonia solanacearum", APPLIED AND ENVIRONMENTAL MICROBIOLOGY, vol. 71, no. 11, 1 November 2005 (2005-11-01), pages 6808-6815, XP055255704, US ISSN: 0099-2240, DOI: 10.1128/AEM.71.11.6808-6815.2005 cited in the application

## Description

### Introduction

Tyrosinase is a type of polyphenol oxidase enzyme widely distributed in nature and has several biological functions and biotechnological applications. In the presence of molecular oxygen, tyrosinase catalyses two types of reaction: the hydroxylation of monophenols to *o-*diphenols (monophenolase activity) and the subsequent oxidation of *o*-diphenols to *o-*quinones (diphenolase activity). The reactive quinones autopolymerize (non-enzymatically) to the macromolecular melanins which are responsible for skin and hair pigmentation, browning of fruit and wound healing in plants and arthropods. Tyrosinase has broad substrate specificity and can accept many types of phenols and diphenols (catechols). Among the substituted phenols it can act on 3- and 4- substituted phenols but 2-substituted phenols are competitive inhibitors of the enzyme.

The ability of tyrosinases to convert monophenols into *o*-diphenols has motivated studies regarding the production of various *o*-diphenols which are important precursors for the synthesis of pharmaceuticals, agrochemicals, flavours, polymerization inhibitors, inks, and antioxidants. Halocatechols are interesting synthons due to their biological activity as well as a variety of cross-coupling and halogen-metal exchange reactions starting from halocatechol intermediates to enable the synthesis of functionalized catechol building blocks. For example, fluorocatechol is potentially a valuable precursor for synthesis of pharmaceuticals, such as the adrenergic catecholamines and biogenic amines. Synthesis of substituted catechol by chemical means is complicated due to the employment of aggressive reagents, severe reaction conditions and poor yield. While tyrosinase has great potential as a biological means of synthesizing these catechols, the use of tyrosinases for catechol synthesis has been limited due to the low ratio of monophenolase to diphenolase activity where the accumulation of catechols is not favoured.

Hydroxytyrosol is a highly desired antioxidant used in food, cosmetics, and medicine. The European Food Safety Authority released a statement in 2011 supporting certain claims made about the health benefits of hydroxytyrosol. The price of hydroxytyrosol is high due to a difficult production process (e.g. extraction from olive leaves, chemical synthesis, and extraction from olive oil mill wastewater). The purity of the majority of hydroxytyrosol containing product is low (<80% and often less than 30%) with phenolics and other contaminants present. The low quality, high cost is limiting the application of hydroxytyrosol. The use of a biocatalyst such as tyrosinase would allow for high quality hydroxytyrosol.

A chemical process for production of hydroxytyrosol from tyrosol has been reported but with only 50% conversion at 6mM concentration (EP1623960). Whole cells of *P. aeruginosa* can transform tyrosol to hydroxytyrosol (80-96% yield at 25 mM) but produces undesired byproducts (p-hydroxyphenylacetic acid and 3,4 dihydroxyphenylacetic acid) which increases the complexity and cost of the downstream process (Allouche et al., Appl. Environ. Microbiol., 2004, 70, pp 2105-2109; Boullagui and Sayadi, J. Agric. Food Chem., 2006, 54, pp 9906-9911) Furthermore *P. aeruginosa* is an opportunistic pathogen which adds cost to biological control procedures within a production facility. It is unlikely to be used to produce food additives such as hydroxytyrosol.

ES2320505 describes a process for obtaining L-DOPA from L-tyrosine using the enzyme tyrosinase NP_518485 from the bacterium *Ralstonia solanacearum.* Molloy et al (Biotechnol. Bioeng. 2013, 110, pp 1849-1857) describes an engineered tyrosine enzyme from *Ralstonia solanacearum* for improved catalytic efficiency towards D-tyrosine using random and site directed mutagenesis.

US2003180833 (D1) describes the bioconversion of tyrosol into hydroxytyrosol using a mushroom-derived tyrosinase enzyme. While the process provides for high yields, the reaction times were very slow, with 1g tyrosol conversion using 15 mg of mushroom tyrosinase in a one litre reaction requiring 5 h to complete the reaction. Moreover, preparations of commercial mushroom tyrosinase are inhibited above 10 g/l of tyrosol and cannot complete the reaction. Scientific literature reports inhibition of mushroom tyrosinase by ascorbic acid at 5 mM (Golan-Goldhirsh and Whitaker, J. Agric. Food Chem., 1984, 32, pp 1003-1009; Marín-Zamora et al, J. Biotechnol., 2009, 139, pp 163-168). The *Ralstonia solanacearum* tyrosinase can act as a biocatalyst as a whole cell, crude cell lysate or purified enzyme. The first two methods of biocatalyst preparation are easy and offer advantages over mushroom tyrosinase used in a purified preparation. It has been suggested that repeated batch system for the conversion of tyrosol (5 g/1) to hydroxytyrosol is possible (Bouallagui and Sayedi J. Agric. Food Chem., 2006, 54 (26), pp 9906-9911) but yields are at 85% in a single run and biocatalyst loses 60% of its activity after 3 runs resulting in poor yields, low overall concentrations and leaving high concentrations of the substrate in the final product. Complicated downstream processing to achieve a highly pure hydroxytyrosol will thus be needed. Furthermore the repeated cycles is cumbersome. Another variation on the fed batch method claims repeated product removal with beads (Brouk and Fishman, J Mol Catal B: Enzym 84:121-127) but this resulted in low product concentration and less than 50% yield.

Brooks et al (Enzyme and Microbial Technology, Vol. 39, No. 2, 26 June 2006 and Applied Microbiology and Biotechnology Vol. 64, 2004, pages 486-492) describe an enzymatic process for converting tyrosol or a halophenol to the corresponding catechol product, and the use of a tyrosinase from *Pseudomonas putida* F6 to catalyse the reaction. Yields for the reaction were 77% using a substrate concentration of 1 mM tyrosol, but were reduced to 5% when the substrate concentration was increased to 2.5 mM tyrosol, due to substrate inhibition.

It is an object of the invention to overcome at least one of the above-referenced problems.

### Statements of Invention

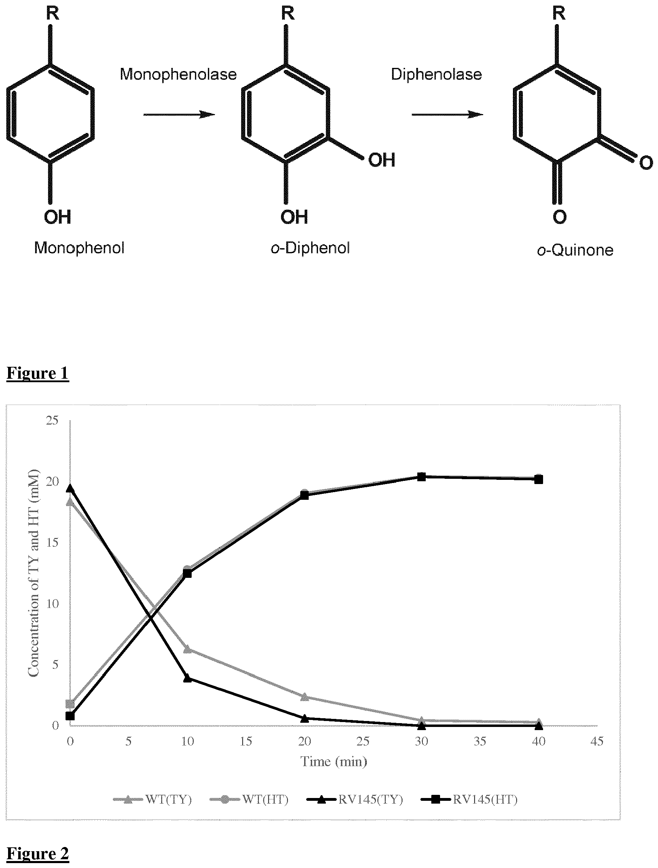

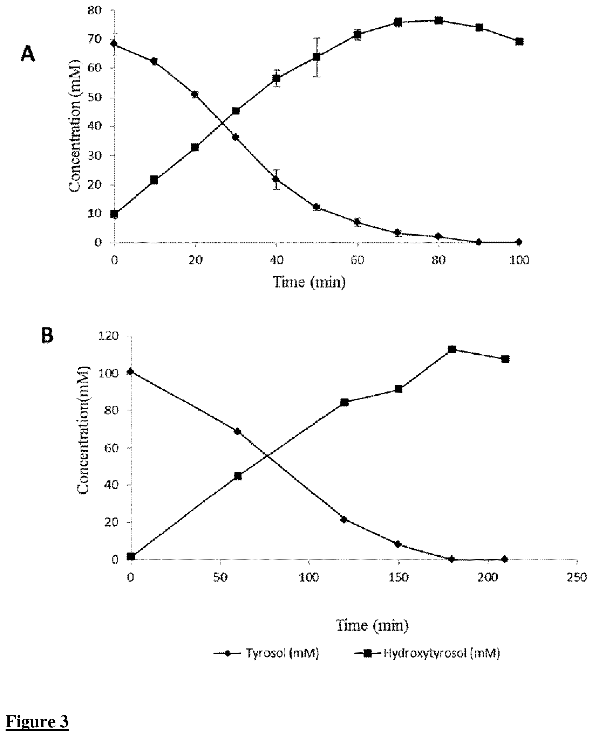

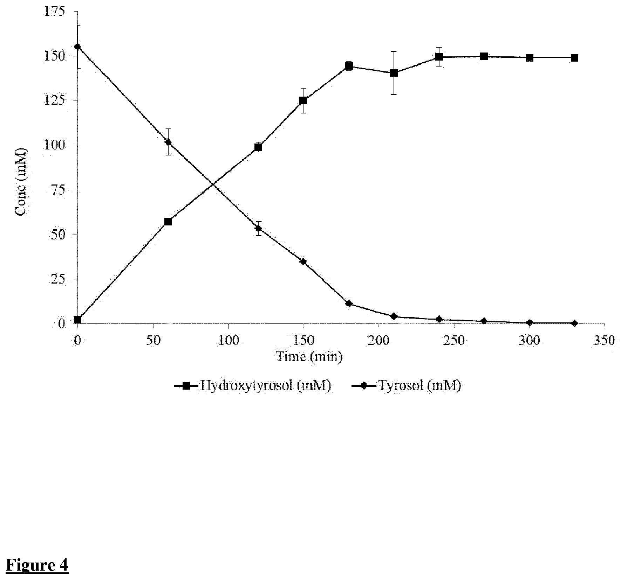

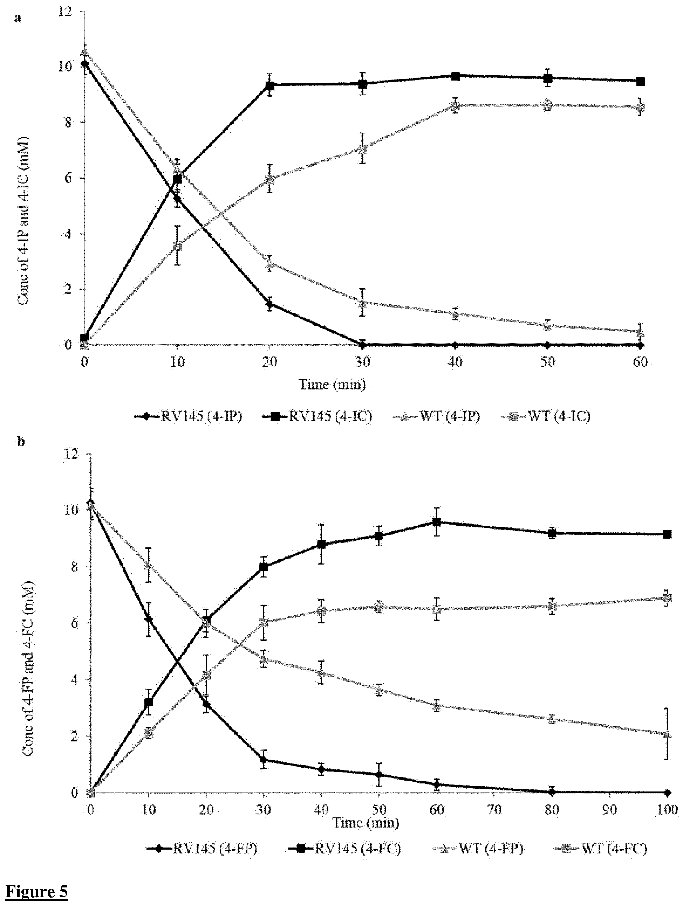

The Applicant has identified a tyrosinase enzyme from *Ralstonia solanacearum* that is capable of converting tyrosol into a hydroxytyrosol at a very high concentration (up to 150mM and rate (up to 9.3 g/l/h), compared to any other biocatalyst (best at 25 mM and 0.4 g/l/h) (Figs 2-4). The conversion rates of tyrosol to corresponding catechols using *Ralstonia solanacearum* tyrosinase enzyme is far higher than the conversion rate for L-tyrosine, the natural substrate. The Applicant has also demonstrated that the *Ralstonia solanacearum* tyrosinase is capable of converting 4-halophenols into corresponding 4-halocatechols with conversion rates 4 g/l/h and product yields (>92%) that are higher than previously reported using tyrosinase or any other enzyme (Figs 5). The method of the present invention requires 12.9 mg of tyrosinase from *R. solanacearum* to complete 1g tyrosol biotransformation in a one litre reaction in 20 min (15 times faster than commercial mushroom tyrosinase). Furthermore the method of the invention demonstrates reactions at high concentration of substrate that heretofore have not been achieved. The *Ralstonia solanacearum* tyrosinase demonstrates high tolerance to substrate (27.6 g/L of tyrosol) and product (30.8 g/L of hydroxytyrosol). No inhibition of *Ralstonia solanacearum* tyrosinase activity by ascorbic acid sodium salt (up to 400mM) was observed. The *Ralstonia solanacearum* tyrosinase can act as a biocatalyst as a whole cell, crude cell lysate or purified enzyme. The first two methods of biocatalyst preparation are easy and offer advantages over mushroom tyrosianse used in a purified preparation.

Accordingly, the invention provides a method for the enzymatic conversion of a phenol substrate into a corresponding catechol product, in which the phenol substrate is selected from tyrosol and a 4-halophenol, the method comprising the steps of incubating the phenol substrate with a *Ralstonia solanacearum* tyrosinase enzyme, or a functional derivative thereof, in a reaction mixture, for a period of time sufficient to allow the enzyme convert at least some of the phenol substrate into the catechol product and in which the reaction mixture comprises ascorbic acid, and in which the functional derivative is an engineered variant of the *Ralstonia solanacearum* tyrosinase enzyme having 1 to 5 amino acid alterations compared with the *Ralstonia solanacearum* tyrosinase enzyme, wherein the or each alteration is selected from insertion, addition, deletion and substitution of an amino acid.

When the phenol substrate is a tyrosol, the catechol product is hydroxytyrosol. When the phenol substrate is a halophenol, the catechol product is a halocatechol.

Preferably, the reaction mixture comprises 100mM to 300mM ascorbic acid. In one embodiment, the reaction mixture comprises up to 400mM ascorbic acid. In one embodiment, the ascorbic acid is provided as a salt, preferably sodium ascorbate.

Suitably, the engineered variant of *Ralstonia solanacearum* tyrosinase enzyme is selected from the group consisting of RVC10, RV145 and C10_N322S, the details of which are described in Molloy et al., Biotechnol. Bioeng. 2013, 110, pp 1849-1857. In one embodiment, the functional derivative is the engineered enzyme RV145.

In one embodiment, the *Ralstonia solanacearum* tyrosinase enzyme, or functional derivative thereof, is provided as a whole cell, crude cell lysate or purified enzyme. In one embodiment, the enzyme is immobilised to a support.

Typically, the substrate concentration is at least 10mM. In one embodiment, the substrate concentration is at least 100mM. In one embodiment, the substrate concentration is at least 150mM. In one embodiment, the substrate concentration is at least 175mM.

Suitably, the method involves a step of separating catechol product from the enzyme. In one embodiment, the separation step comprises a membrane filtration step. In one embodiment, the separation step comprises a solvent extraction step. In one embodiment the separation is performed using a solid phase to bind and extract the catechol product from the aqueous biotransformation medium.

In one embodiment, the enzyme converts 90% of the phenol substrate into the catechol product. In one embodiment, the enzyme converts 100% of the phenol substrate into the catechol product.

The invention also relates to a catechol product selected from hydroxytyrosol and a 4-halocatechol made according to the method of the invention.

### Brief Description of the Figures

**Fig. 1****:** Hydroxylation of halophenols by the monophenolase activity and oxidation of halocatechols by the diphenolase activity of tyrosinase
**Fig 2**:Biotransformation of 20 mM of tyrosol in shaking flasks using WT and RV145 enzymes (20 µg/mL) in the presence of 40 mM ascorbic acid sodium salt (TY: tyrosol, HT: Hydroxytyrosol).
**Fig. 3****:** Biotransformation of tyrosol to hydroxytyrosol by engineered RV145 enzyme. a: 75 mM biotransformation of tyrosol using purified engineered RV145 enzyme in the presence of 150mM ascorbic acid sodium salt. B: 100mM biotransformation of tyrosol using cell free lysate of *E. coli* harbouring engineered RV145 enzyme in the presence of 200mM ascorbic acid sodium salt
**Fig. 4****:** 150mM biotransformation of tyrosol using crude cell free lysate of *E. coli* harbouring engineered RV145 enzyme in the presence of 300 mM ascorbic acid sodium salt
**Fig. 5a****:** Biotransformation of 10 mM of 4-fluorophenol in shaking flasks using WT and RV145 enzymes (20 µg/mL) in the presence of 20 mM ascorbic acid sodium salt. **b** Biotransformation of 10 mM of 4-iodophenol in shaking flasks using WT and RV145 enzymes (10µg/mL) in the presence of 20 mM ascorbic acid sodium salt (4-IP: 4-iodocphenol, 4-IC: 4-iodocatechol, 4-FP: 4-fluorophenol, 4-FC: 4-fluorocatechol)

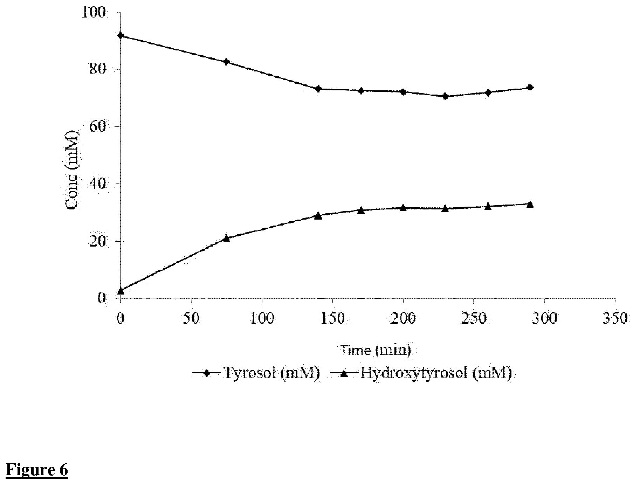

**Figure 6****:** 100mM biotransformation of tyrosol using commercial mushroom tyrosinase in the presence of 200 mM ascorbic acid. The reaction yields only 30% product and stops once 30 mM product is formed showing its much poorer performance compared to *R. solanacearum* tyrosinase.

### Detailed Description of the Invention

### Definitions:

"Phenol substrate" means an unsubstituted or substituted phenol, especially a 3-substituted or 4-substituted phenol. Examples of substituents include halogens or hydroxyalkyl substituents. Examples of hydroxyalkyl substituents include hydroxymethyl and hydroxyethyl substituents, especially 4-hydroxymethyl and 4-hydroxyethyl substituents. Typically, the 3-substituted or 4-substituted phenol is a 3-halophenol or 4-halophenol. Examples of halophenols include iodophenol, bromophenol, chlorophenol, and fluorophenol. Preferably, the phenol is tyrosol. Preferably, the phenol substrate is provided at a concentration up to their maximum solubility in aqueous environment. In one embodiment, the phenol substrate is selected from tyrosol, a 3-halophenol, and a 4-halophenol. In one embodiment, the phenol substrate is selected from tyrosol and a 4-halophenol.

"Corresponding catechol product" means a catechol that is produced by reacting a phenol substrate with a *Ralstonia solanacearum* tyrosinase enzyme. When the phenol substrate is a 4-halophenol (for example, 4-fluorophenol), the corresponding catechol product is the corresponding 4-fluorocatechol (4-fluorocatechol). Likewise, when the phenol substrate is 4-substituted hydroxyalkyl phenol (for example, tyrosol), the corresponding catechol product is the corresponding 4-substituted hydroxyalkyl catechol (hydroxytyrosol).

"Tyrosol" refers to 4-(2-hydroxyethyl)phenol.

"Hydroxytyrosol" means 4-(2-hydroxyethyl)-1,2-benzenediol.

"*Ralstonia solanacearum* tyrosinase enzyme" means a tyrosinease enzyme isolated from *Ralstonia solanacearum.* An example of such an enzyme is described in Molloy et al., Biotechnol. Bioeng. 2013, 110, pp 1849-1857. The amino acid and nucleic acid sequences for the wild-type enzyme are provided below:
Ralstonia solanacearum tyrosinase enzyme amino acid sequence (SEQ ID NO: 1) (NP_518458) 
MVVRRTVLKAIAGTSVATVFAGKLTGLSAVAADAAPLRVRRNLHGMKMDDPDLSAYREFVGIMKGKDQTQ
ALSWLGFANQHGTLNGGYKYCPHGDWYFLPWHRGFVLMYERAVAALTGYKTFAMPYWNWTEDRLLPEAFT
AKTYNGKTNPLYVPNRNELTGPYALTDAIVGQKEVMDKIYAETNFEVFGTSRSVDRSVRPPLVQNSLDPK
WVPMGGGNQGILERTPHNTVHNNIGAFMPTAASPRDPVFMMHHGNIDRVWATWNALGRKNSTDPLWLGMK
FPNNYIDPQGRYYTQGVSDLLSTEALGYRYDVMPRADNKVVNNARAEHLLALFKTGDSVKLADHIRLRSV
LKGEHPVATAVEPLNSAVQFEAGTVTGALGADVGTGSTTEVVALIKNIRIPYNVISIRVFVNLPNANLDV
PETDPHFVTSLSFLTHAAGHDHHALPSTMVNLTDTLKALNIRDDNFSINLVAVPQPGVAVESSGGVTPES
IEVAVI
*Ralstonia solanacearum* tyrosinase enzyme nucleic acid sequence (SEQ ID NO. 2) (gi|30407127)

TCAAATGACGGCGACCTCGATCGATTCGGGCGTCACGCCGCCGCTGCTCTCCACGGCAACGCCGGGTTGG
GGTACGGCCACCAGGTTGATCGAAAAGTTGTCGTCCCGGATGTTGAGCGCCTTCAGCGTGTCGGTCAGGT
TCACCATGGTCGACGGCAGGGCATGGTGGTCGTGTCCCGCCGCATGCGTCAGGAAGCTGAGCGAGGTGAC
GAAGTGCGGGTCGGTTTCCGGCACATCGAGGTTGGCGTTCGGCAGGTTGACGAAGACCCGGATGCTGATC
ACGTTGTAGGGGATCCTGATGTTCTTGATCAGGGCCACGACTTCGGTGGTACTGCCGGTACCAACATCGG
CACCCAGGGCACCCGTCACGGTGCCGGCCTCGAACTGGACGGCGCTGTTGAGCGGTTCGACCGCCGTGGC
AACCGGATGTTCCCCCTTCAGCACGCTGCGCAGCCGGATATGATCGGCCAGCTTGACGCTGTCGCCGGTC
TTGAACAGGGCCAGCAGATGCTCGGCACGGGCGTTGTTCACCACCTTGTTGTCGGCGCGCGGCATGACGT
CATAGCGGTAGCCCAGCGCCTCGGTGCTCAGCAGATCGCTCACGCCTTGCGTGTAGTACCGGCCCTGCGG
ATCGATGTAGTTGTTGGGGAACTTCATGCCCAGCCACAGCGGGTCAGTCGAGTTCTTGCGGCCCAGCGCG
TTCCAGGTGGCCCATACCCGGTCGATATTGCCGTGGTGCATCATGAACACCGGGTCGCGCGGCGAGGCGG
CGGTGGGCATGAAGGCGCCGATGTTGTTGTGGACGGTGTTGTGCGGCGTGCGCTCCAGGATGCCCTGGTT
GCCGCCTCCCATCGGCACCCATTTGGGGTCGAGGCTGTTCTGTACCAGCGGCGGCCGGACCGAGCGGTCG
ACCGAACGGCTGGTGCCGAAGACTTCGAAGTTGGTTTCGGCATAGATCTTGTCCATGACCTCCTTCTGGC
CGACGATGGCGTCGGTGAGCGCGTAGGGGCCGGTCAGCTCATTCCGGTTGGGCACGTAGAGCGGGTTCGT
CTTGCCGTTGTAGGTCTTGGCGGTGAAGGCTTCGGGCAGCAGGCGGTCTTCGGTCCAGTTCCAGTACGGC
ATGGCGAAGGTCTTGTAGCCGGTGAGCGCGGCCACGGCGCGCTCGTACATCAGCACGAAGCCGCGGTGCC
AGGGCAGGAAGTACCAGTCGCCGTGCGGGCAGTACTTGTAGCCGCCGTTGAGCGTACCGTGCTGGTTGGC
AAAGCCGAGCCAGCTCAGCGCCTGCGTCTGGTCCTTGCCTTTCATGATGCCGACGAACTCGCGATAGGCC
GACAGGTCCGGGTCGTCCATCTTCATGCCATGCAGGTTGCGCCGCACGCGCAGCGGGGCGGCATCGGCCG
CAACAGCGGAGAGGCCGGTCAGCTTGCCCGCGAATACCGTGGCGACACTTGTCCCGGCGATTGCCTTCAG
CACCGTTCTACGCACGACCAT

"Functional derivative thereof" as applied to a *Ralstonia solanacearum* tyrosinase enzyme means an engineered variant of *Ralstonia solanacearum* tyrosinase enzyme that is typically capable of converting a phenol (i.e. tyrosol) into a corresponding catechol (i.e.hydroxytyrosol) at a concentration and rate that is significantly better than mushroom tyrosinease described in US2003180833 (D1). Examples of such engineered variants of *Ralstonia solanacearum* tyrosinase enzyme are described in Molloy et al (2013), including variants that are capable of converting tyrosol into hydroxytyrosol at a concentration and rate that is at least the equivalent of the wild-type *Ralstonia solanacearum* tyrosinase enzyme. In one embodiment, the engineered variant of *Ralstonia solanacearum* tyrosinase enzyme is capable of the 100% conversion of 150mM tyrosol into hydroxytyrosol in the tyrosol biotransformation assay described below. In one embodiment, the engineered variant of *Ralstonia solanacearum* tyrosinase enzyme is capable of the 100% conversion of 175mM tyrosol into hydroxytyrosol in the tyrosol biotransformation described below. Methods for generating and testing engineered variants of *Ralstonia solanacearum* tyrosinase enzyme will be apparent to the person skilled in the art, and are described in Molloy et al..

An "engineered variant" of the *Ralstonia solanacearum* tyrosinase enzyme protein shall be taken to mean enzymes having amino acid sequences which are substantially identical to wild-type *Ralstonia solanacearum* tyrosinase enzyme. Thus, for example, the term should be taken to include enzymes that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The engineered variant may have conservative amino acid changes, wherein the amino acid being introduced is similar structurally, chemically, or functionally to that being substituted. Generally, the variant will have at least 70% amino acid sequence homology, preferably at least 80% sequence homology, more preferably at least 90% sequence homology, and ideally at least 95%, 96%, 97%, 98% or 99% sequence homology with wild-type *Ralstonia solanacearum* tyrosinase enzyme. In this context, sequence homology comprises both sequence identity and similarity, i.e. a polypeptide sequence that shares 70% amino acid homology with wild-type *Ralstonia solanacearum* tyrosinase enzyme is one in which any 70% of aligned residues are either identical to, or conservative substitutions of, the corresponding residues in wild-type *Ralstonia solanacearum* tyrosinase enzyme. Specific variants included within the scope of the invention are the engineered variants described in (Molloy et al., 2013), especially (RVC10, RV145 and C10_N322S). In one embodiment, the engineered variant of *Ralstonia solanacearum* tyrosinase enzyme is capable of complete conversion of a tyrosol substrate to hydroxytyrosol at concentrations an order of magnitude higher than previously reported by any tyrosinase or other biocatalyst (e.g. 175 mM tyrosol)

The term "engineered variant" is also intended to include chemical derivatives of wild-type *Ralstonia solanacearum* tyrosinase enzyme, i.e. where one or more residues of the wild-type enzyme is chemically derivatized by reaction of a functional side group. Also included within the term variant are wild-type *Ralstonia solanacearum* tyrosinase enzymes in which naturally occurring amino acid residues are replaced with amino acid analogues. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during enzyme synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

### EXPERIMENTAL

### Materials and methods

### Strains, growth conditions and purification of tyrosinase

Recombinant *E. coli* BL21 were used to express wild type (WT) tyrosinase gene from *R*. *solanacearum* and three engineered variants (RV145, RVC10, C10_N322S) of the same gene previously generated by Molloy et al (2013). All strains were maintained in Lysogeny broth (LB) with 50% glycerol at ⁻80°C. In preparation for induction and tyrosinase purification, the *E. coli* strains stored at ⁻80°C were streaked on LB agar and incubated at 37°C for 24 h. The primary inoculum was cultured in a rotary shaker (200 rpm, 37°C) in 5 mL of LB overnight. Carbenicillin (50µg/mL) was used as the antibiotic for the maintenance of the plasmid throughout the study. Tyrosinase production was induced and the enzyme was purified as described earlier (Molloy et al. 2013). Different fractions of the purified tyrosinase were analysed by 10 % SDS-PAGE for confirmation of purity and molecular weight. The protein concentration was determined by bicinchoninic acid method (Smith et al. 1985) using bovine serum albumin as the standard.

### Preparation of substrates and other chemicals

Various monophenol substrates such as 4-fluorophenol, 4-bromophenol, 4-chlorophenol, and 4-iodophenol were purchased from Sigma-Aldrich (Dublin, Ireland). Stock solutions (1 M) of the 4-bromophenol, and 4-iodophenol were prepared in 100% ethanol and subsequent working solutions were further diluted in 50 mM potassium phosphate buffer (pH 7). Similarly, stock solutions (500 mM) of 4-fluorophenol and 4-chlorophenol were prepared in deionized water and working stocks were prepared in 50 mM phosphate buffer.. Tyrosol was purchased from TCI Europe (Belgium) and stocks were prepared in 50 mM phosphate buffer. Ascrobic acid and ascorbic acid sodium salt (AA) (used to reduce o-quinone to o-diphenol) was prepared in deionized water freshly before the biotransformation assay

### Biotransformation of 4-halogenated phenols and tyrosol

Biotransformations of halophenols into corresponding halocatechols were carried out using purified enzymes (WT and engineered variants). Various concentrations (5, 10, and 20 mM) of halophenol biotransformations were carried out in 100 mL conical flasks with a working volume of 20 mL at 30°C and 200 rpm in a shaker incubator. Biotransformation of tyrosol to hydroxytyrosol was carried out using purified enzymes, cell free lysate or whole cells. Various concentrations (75, 100, 150 and 175 mM) of tyrosol biotransformations were carried out in 500 mL baffled conical flasks with a working volume of 100 mL at 30°C and 200-250 rpm in a shaker incubator. An enzyme concentration of 2 µg/mL per 1 mM of substrate was tested with a substrate to sodium ascorbate ratio of 1:2. Biotransformation of 100mM tyrosol to hydroxytyrosol was also carried out using commercial mushroom tyrosinase (Sigma, Ireland) using the above specified conditions. Samples (450 µL) were withdrawn at various time points and immediately added to 50 µL of ice-cold IN HCl. Samples were centrifuged (12,000 g), filtered using Whatman Mini- UniPrep™ syringeless filters (0.45µ Whatman Inc. NJ, USA), and a sample volume of 20 µL was used for all HPLC injections.

### HPLC analysis

Filtered samples were analyzed by HPLC using a 5 µm ACE 5 C18column (25 cm× 4.6 mm ID; Apex Scientific, Ireland) and a Hewlett-Packard (Palo Alto, CA, USA) HP1100 instrument equipped with an Agilent 1100 Series Diode Array Detector. The samples were isocratically eluted at 22°C using a phosphoric acid (0.1 %, v/v) and methanol mix at a flow rate of 1.0 mL/min. The ratio of phosphoric acid to methanol was 70:30 for 4-fluorophenol and 50:50 for 4-chloro-, 4-bromo-, and 4-iodophenol.

## Claims

1. A method for the enzymatic conversion of a phenol substrate into a corresponding catechol product, in which the phenol substrate is selected from tyrosol and a 4-halophenol, the method comprising the steps of incubating the phenol substrate with a *Ralstonia solanacearum* tyrosinase enzyme, or a functional derivative thereof, in a reaction mixture, for a period of time sufficient to allow the enzyme convert at least some of the phenol substrate into the catechol product and in which the reaction mixture comprises ascorbic acid, in which the functional derivative is an engineered variant of the *Ralstonia solanacearum* tyrosinase enzyme having 1 to 5 amino acid alterations compared with the *Ralstonia solanacearum* tyrosinase enzyme, wherein the or each alteration is selected from insertion, addition, deletion and substitution of an amino acid.

2. A method according to Claim 1 in which the phenol substrate is a tyrosol and the catechol product is hydroxytyrosol.

3. A method according to Claim 1 in which the phenol substrate is a halophenol and the catechol product is a halocatechol.

4. A method according to any preceding Claim in which the ascorbic acid is provided as a salt.

5. A method according to any preceding Claim in which the engineered variant of *Ralstonia solanacearum* tyrosinase enzyme is capable of 100% conversion of 175mM tyrosol into hydroxytyrosol

6. A method according to any preceding Claim in which the engineered variant of *Ralstonia solanacearum* tyrosinase enzyme is selected from the group consisting of RVC10, RV145 and C10_N322S.

7. A method according to any preceding Claim in which the *Ralstonia solanacearum* tyrosinase enzyme, or functional derivative thereof, is provided as an extract from a bacteria that expresses the *Ralstonia solanacearum* tyrosinase enzyme, or functional derivative thereof.

8. A method according to any of Claims 1 to 7 in which the *Ralstonia solanacearum* tyrosinase enzyme, or functional derivative thereof, is provided as a bacteria that expresses the *Ralstonia solanacearum* tyrosinase enzyme, or functional derivative thereof.

9. A method according to any of Claims 1 to 6 in which the *Ralstonia solanacearum* tyrosinase enzyme, or functional derivative thereof, is provided as a purified enzyme.

10. A method according to any of Claims 1 to 6 in which the *Ralstonia solanacearum* tyrosinase enzyme, or functional derivative thereof, is provided as a crude mixture in the form of a cell extract.

11. A method according to any preceding Claim, in which the substrate concentration is at least 75mM.

12. A method as claimed in any preceding Claim in which the catechol product is separated from impurities present in the reaction mixture by chilling the reaction mixture after the enzymatic conversion step.

13. A method as claimed in any preceding Claim in which the catechol product is separated from impurities present in the reaction mixture by refrigerating or freezing the reaction mixture after the enzymatic conversion step.

## Patentansprüche

1. Verfahren für die enzymatische Umwandlung eines Phenolsubstrats in ein entsprechendes Catecholprodukt, in dem das Phenolsubstrat aus Tyrosol und einem 4-Halogenphenol ausgewählt ist, wobei das Verfahren die Schritte des Inkubierens des Phenolsubstrats mit einem Tyrosinase-Enzym von *Ralstonia solanacearum* oder einem funktionellen Derivat davon in einer Reaktionsmischung für eine Zeitdauer umfasst, die ausreicht, dem Enzym die Umwandlung von mindestens etwas des Phenolsubstrats in das Catecholprodukt zu erlauben, und in dem die Reaktionsmischung Ascorbinsäure umfasst, in dem das funktionelle Derivat eine konstruierte Variante des Tyrosinase-Enzyms von *Ralstonia solanacearum* ist, die 1 bis 5 Aminosäureänderungen aufweist im Vergleich zum Tyrosinase-Enzym von *Ralstonia solanacearum*, wobei die oder jede Änderung aus Insertion, Addition, Deletion und Substitution einer Aminosäure ausgewählt ist.

2. Verfahren nach Anspruch 1, in dem das Phenolsubstrat ein Tyrosol ist und das Catecholprodukt Hydroxytyrosol ist.

3. Verfahren nach Anspruch 1, in dem das Phenolsubstrat ein Halogenphenol ist und das Catecholprodukt ein Halogencatechol ist.

4. Verfahren nach einem vorstehenden Anspruch, in dem die Ascorbinsäure als ein Salz bereitgestellt wird.

5. Verfahren nach einem vorstehenden Anspruch, in dem die konstruierte Variante des Tyrosinase-Enzyms von *Ralstonia solanacearum* zu 100 % Umwandlung von 175 mM Tyrosol in Hydroxytyrosol fähig ist.

6. Verfahren nach einem vorstehenden Anspruch, in dem die konstruierte Variante des Tyrosinase-Enzyms von *Ralstonia solanacearum* aus der Gruppe ausgewählt ist, die aus RVC10, RV145 und C10_N322S besteht.

7. Verfahren nach einem vorstehenden Anspruch, in dem das Tyrosinase-Enzym von *Ralstonia solanacearum* oder funktionelle Derivat davon als Extrakt aus einem Bakterium bereitgestellt wird, das das Tyrosinase-Enzym von *Ralstonia solanacearum* oder funktionelle Derivat davon exprimiert.

8. Verfahren nach einem der Ansprüche 1 bis 7, in dem das Tyrosinase-Enzym von *Ralstonia solanacearum* oder funktionelle Derivat davon als ein Bakterium bereitgestellt wird, das das Tyrosinase-Enzym von *Ralstonia solanacearum* oder funktionelle Derivat davon exprimiert.

9. Verfahren nach einem der Ansprüche 1 bis 6, in dem das Tyrosinase-Enzym von *Ralstonia solanacearum* oder funktionelle Derivat davon als ein aufgereinigtes Enzym bereitgestellt wird.

10. Verfahren nach einem der Ansprüche 1 bis 6, in dem das Tyrosinase-Enzym von *Ralstonia solanacearum* oder funktionelle Derivat davon als eine Rohmischung in Form eines Zellextrakts bereitgestellt wird.

11. Verfahren nach einem vorstehenden Anspruch, in dem die Substratkonzentration mindestens 75 mM beträgt.

12. Verfahren nach einem vorstehenden Anspruch, in dem das Catecholprodukt von Verunreinigungen, die in der Reaktionsmischung vorliegen, durch Abkühlen der Reaktionsmischung nach dem enzymatischen Umwandlungsschritt abgetrennt wird.

13. Verfahren nach einem vorstehenden Anspruch, in dem das Catecholprodukt von Verunreinigungen, die in der Reaktionsmischung vorliegen, durch Kühlen oder Einfrieren der Reaktionsmischung nach dem enzymatischen Umwandlungsschritt abgetrennt wird.

## Revendications

1. Méthode destinée à la conversion enzymatique d'un substrat de phénol en un produit de catéchol correspondant, dans laquelle le substrat de phénol est choisi parmi le tyrosol et un 4-halogénophénol, la méthode comprenant les étapes consistant à incuber le substrat de phénol avec une enzyme de type tyrosinase de *Ralstonia solanacearum*, ou un dérivé fonctionnel de celle-ci, dans un mélange réactionnel, pendant une période de temps suffisante pour permettre à l'enzyme de convertir au moins une partie du substrat de phénol en produit de catéchol et où le mélange réactionnel comprend de l'acide ascorbique, où le dérivé fonctionnel est un variant modifié de l'enzyme de type tyrosinase de *Ralstonia solanacearum* ayant de 1 à 5 altérations d'acides aminés par rapport à l'enzyme de type tyrosinase de *Ralstonia solanacearum*, où l'altération, ou chacune d'entre elles, est choisie parmi une insertion, une addition, une délétion et une substitution d'un acide aminé.

2. Méthode selon la revendication 1, dans laquelle le substrat de phénol est un tyrosol et le produit de catéchol est l'hydroxytyrosol.

3. Méthode selon la revendication 1, dans laquelle le substrat de phénol est un halogénophénol et le produit de catéchol est un halogénocatéchol.

4. Méthode selon l'une quelconque des revendications précédentes, dans laquelle l'acide ascorbique est fourni sous forme de sel.

5. Méthode selon l'une quelconque des revendications précédentes, dans laquelle le variant modifié de l'enzyme de type tyrosinase de *Ralstonia solanacearum* est capable d'effectuer une conversion de 100% de 175 mM de tyrosol en hydroxytyrosol.

6. Méthode selon l'une quelconque des revendications précédentes, dans laquelle le variant modifié de l'enzyme de type tyrosinase de *Ralstonia solanacearum* est choisi dans le groupe constitué par RVC10, RVC145 et C10_N322S.

7. Méthode selon l'une quelconque des revendications précédentes, dans laquelle l'enzyme de type tyrosinase de *Ralstonia solanacearum*, ou un dérivé fonctionnel de celle-ci, est fourni(e) sous forme d'un extrait issu d'une bactérie qui exprime l'enzyme de type tyrosinase de *Ralstonia solanacearum*, ou un dérivé fonctionnel de celle-ci.

8. Méthode selon l'une quelconque des revendications 1 à 7, dans laquelle l'enzyme de type tyrosinase de *Ralstonia solanacearum*, ou un dérivé fonctionnel de celle-ci, est fourni(e) sous forme d'une bactérie qui exprime l'enzyme de type tyrosinase de *Ralstonia solanacearum*, ou un dérivé fonctionnel de celle-ci.

9. Méthode selon l'une quelconque des revendications 1 à 6, dans laquelle l'enzyme de type tyrosinase de *Ralstonia solanacearum*, ou un dérivé fonctionnel de celle-ci, est fourni(e) sous forme d'une enzyme purifiée.

10. Méthode selon l'une quelconque des revendications 1 à 6, dans laquelle l'enzyme de type tyrosinase de *Ralstonia solanacearum*, ou un dérivé fonctionnel de celle-ci, est fourni(e) comme mélange brut sous forme d'un extrait cellulaire.

11. Méthode selon l'une quelconque des revendications précédentes, dans laquelle la concentration en substrat est d'au moins 75 mM.

12. Méthode selon l'une quelconque des revendications précédentes, dans laquelle le produit de catéchol est séparé des impuretés présentes dans le mélange réactionnel par le refroidissement du mélange réactionnel après l'étape de conversion enzymatique.

13. Méthode selon l'une quelconque des revendications précédentes, dans laquelle le produit de catéchol est séparé des impuretés présentes dans le mélange réactionnel par la réfrigération ou la congélation du mélange réactionnel après l'étape de conversion enzymatique.
